# EUROPEAN PATENT SPECIFICATION

(11) **EP 3 156 788 B1**
(45) Date of publication and mention of the grant of the patent: **26.12.2018**
(21) Application number: 16194027.5
(22) Date of filing: 14.10.2016
(51) Int. Cl.: G01N 27/327, C12Q 1/00

(54) **BIOSENSOR**
BIOSENSOR
BIOCAPTEUR

(30) Priority: 15.10.2015 JP 2015204034; 05.10.2016 JP 2016197098
(43) Date of publication of application: 19.04.2017
(73) Proprietor: ARKRAY, Inc., Minami-ku Kyoto-shi, Kyoto 601-8045 (JP); Ultizyme International Ltd., Meguro-ku, Tokyo 152-0013 (JP)
(72) Inventor: SAEDA, Megumi, Kyoto-shi, Kyoto 602-0008 (JP); SHIMAZAKI, Junko, Meguro-ku, Tokyo 152-0013 (JP)
(74) Representative: Dehns

(56) References cited:
- WO-A1-95/28634
- US-A1- 2009 321 277
- Allen Bard ET AL: "Reference electrodes" In: "ELECTROCHEMICAL METHODS", 1 January 2001 (2001-01-01), John Wiley & Sons, USA, XP055342847, pages 53-54, * the whole document *

## Description

### FIELD

The embodiment pertains to a biosensor for measuring a charge transfer limiting current.

### BACKGROUND

There exists an enzyme electrode including an electrode as a base material, and a detection layer configured by solidifying enzymes and conductive particles on a surface of the electrode by using a crosslinking agent and a binder. The enzyme electrode has a structure of transferring and receiving electrons generated by enzymatic reaction. The enzyme electrode exists, which includes the detection layer containing the enzymes, the conductive particles and the crosslinking agent (e.g., Patent document 1). Another enzyme electrode exists, which includes the detection layer containing the enzymes, the conductive particles and electrically conductive polymers (e.g., Patent document 2).
[Patent document 1] Japanese Laid-open Patent Publication No. 2014-006154
[Patent document 2] Japanese Laid-open Patent Publication No. 2014-006155

### SUMMARY

In the enzyme electrodes of Patent document 1 and Patent document 2, a silver-silver chloride (Ag/AgCl) electrode is used as a reference electrode. The silver-silver chloride electrode has a problem that an electrode property is deteriorated when oxidated. It is an object of the embodiment, which is devised under such circumstances, to provide a biosensor capable of restraining the electrode property from being deteriorated.

According to an aspect of the embodiment, a biosensor includes: a working electrode; a counter electrode; a reference electrode; and a detection layer contacting the working electrode and containing a crosslinking agent, an electrically conductive polymer, and an enzyme transferring and receiving electrons to and from the working electrode, the reference electrode being a polarizable electrode which is carbon or gold.

The working electrode and the counter electrode may be polarizable electrodes, and the working electrode, the counter electrode and the reference electrode may be made from the same material.

According to the embodiment, it is feasible to provide the biosensor capable of restraining the electrode property from being deteriorated.

### BRIEF DESCRIPTION OF DRAWINGS

FIG. 1 is a schematic view of a biosensor according to an embodiment;
FIG. 2 is a schematic diagram of an enzyme electrode according to the embodiment;
FIG. 3 is a diagram illustrating one example of a configuration of a measurement apparatus according to the embodiment;
FIG. 4 is a flowchart illustrating one example of a process of measuring a concentration of a measurement target substance by a control computer;
FIG. 5 is a graph indicating a measurement result in Test 1;
FIG. 6 is a graph indicating a measurement result in Comparative Example 1;
FIG. 7 is a graph indicating the measurement results of Test 1 and Comparative Example 1;
FIG. 8 is a graph indicating a measurement result in Test 2;
FIG. 9 is a graph indicating a measurement result in Comparative Example 2; and
FIG. 10 is a graph indicating the measurement results of Test 2 and Comparative Example 2.

### DESCRIPTION OF EMBODIMENT

An embodiment of the present invention will hereinafter be described with reference to the drawings. The embodiment to be hereinafter demonstrated is an exemplification, and the present invention is not limited to a configuration of the embodiment that follows.

### (Biosensor)

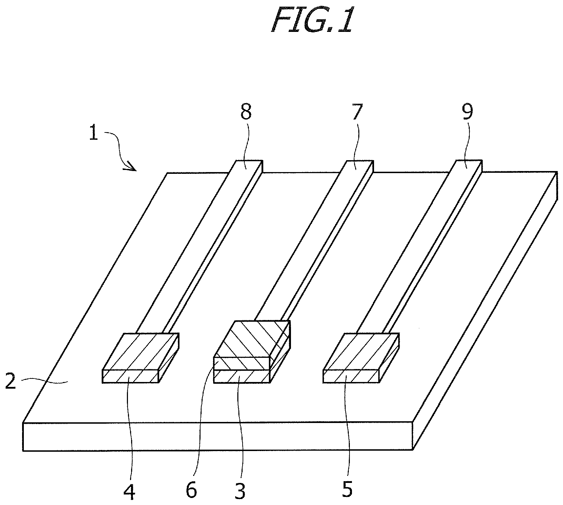

FIG. 1 is a schematic view of a biosensor 1 according to the embodiment, illustrating the biosensor 1 that is partially enlarged. As depicted in FIG. 1, the biosensor 1 includes a substrate 2, a working electrode 3, a counter electrode 4, a reference electrode 5, and a detection layer 6. The working electrode 3, the counter electrode 4 and the reference electrode 5 are formed on an upper surface of the substrate 2. The detection layer 6 is formed on the working electrode 3. The biosensor 1 further includes an unillustrated spacer and an unillustrated cover. The spacer is provided on the substrate 2, and the cover is provided on the spacer, thereby configuring a capillary within the biosensor 1. A lead wire 7 is electrically connected to the working electrode 3, a lead wire 8 is electrically connected to the counter electrode 4, and a lead wire 9 is electrically connected to the reference electrode 5. The lead wires, 7, 8 and 9 are electrically connected to terminals of a measurement apparatus to be described later on.

The substrate 2, the spacer and the cover are composed of insulating materials instanced by a thermoplastic resin, a polyimide resin, an epoxy resin, glass, ceramic and paper. The thermoplastic resin encompasses polyether-imide (PEI), polyethylene terephthalate (PET), polyethylene (PE) and other equivalent materials. All of known materials are applicable as materials of the substrate 2, the spacer and the cover. Dimensions of sizes, thicknesses and other equivalents of the substrate 2, the spacer and the cover are enabled to be properly set

The working electrode 3 and the counter electrode 4 are configured by using metallic materials instanced by gold (Au), platinum (Pt), palladium and ruthenium, or carbonic materials instanced by carbon. The reference electrode is carbon or gold. These electrode materials have a property, i.e., polarity. Accordingly, the working electrode 3, the counter electrode 4 and the reference electrode 5 are polarizable electrodes. The polarizable electrode is an electrode that is easy to be polarized (which means that an electric potential changes) depending on an external equipment and other equivalent devices within a certain potential range. By contrast, a non-polarizable electrode is an electrode that is hard to be polarized depending on the external equipment and other equivalent devices because of causing a large amount of electric current to flow upon being polarized. A silver-silver chloride electrode is one example of the non-polarizable electrode. It has been known so far that the reference electrode involves using the non-polarizable electrode, and the invention of the present application has, however, found out as a result of making assiduous researches that the polarized electrode is usable as the reference electrode. The biosensor 1 according to the embodiment uses the polarizable electrode made from carbon or gold as the reference electrode 5 but does not use the silver-silver chloride electrode as the reference electrode 5. The electrode (polarizable electrode) configured by using the metallic materials gold or carbon, is higher in resistance against oxidation than the silver-silver chloride electrode. The electrode (polarizable electrode) configured by using the metallic materials gold or carbon, is therefore more restrained from being deteriorated in electrode property than the silver-silver chloride electrode.

The materials of the working electrode 3, the counter electrode 4 and the reference electrode 5 may be the same and may also be different from each other. For example, the working electrode 3 and the counter electrode 4 may be configured by using any one of gold, platinum, palladium, ruthenium and carbon. The reference electrode 5 is configured using gold or carbon. For instance, the working electrode 3 and the counter electrode 4 may be configured by using gold, while the reference electrode 5 may be configured by using carbon; and alternatively, the working electrode 3 and the counter electrode 4 may be configured by using carbon, while the reference electrode 5 may be configured by using gold. In addition, the working electrode 3 and the counter electrode 4 may be configured by using other transition metal.

### (Configuration of Enzyme Electrode)

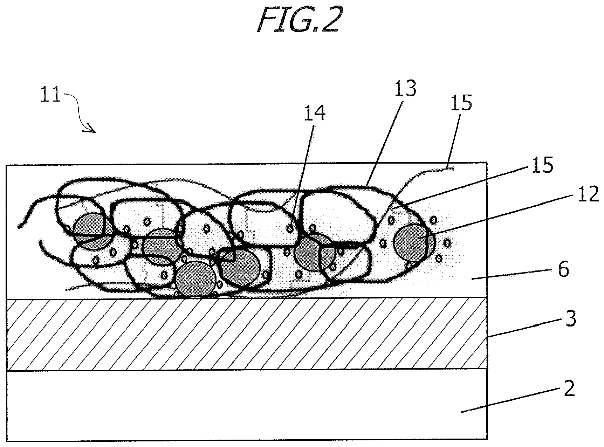

FIG. 2 is a schematic view of an enzyme electrode according to the embodiment. In FIG. 2, an enzyme electrode 11 includes the working electrode 3 and the detection layer 6 formed on the surface (upper surface in FIG. 1) of the working electrode 3.

### (Detection Layer)

The detection layer 6 contacts the working electrode 3, and contains enzymes 12, electrically conductive polymers 13, sugars 14 and a crosslinking agent 15, but does not contain electronic mediators.

The enzyme electrode 11 according to the embodiment is used to measure a charge transfer limiting current based on the transfer of electrons derived from a measurement target substance (a substance to be measured) to the electrode. The charge transfer limiting current is a current which is generated when the electrons are transferred from the enzymes 12 to the electrode due to the reaction between the enzymes 12 and the measurement target substance . Further, the charge transfer limiting current is a steady-state current which does not depend on time, and preferably, a steady-state current observed after the generation of a transient current due to the charging of an electric double layer.

In order to measure the charge transfer limiting current, it is preferred that a "direct electron transfer-type enzyme electrode" be used as the working electrode 3. The "direct electron transfer-type enzyme electrode" as used herein refers to a type of an enzyme electrode in which electrons are transferred between the enzyme and the electrode in such a way that electrons generated by an enzyme reaction in a reagent layer are directly transferred to the electrode (including the case in which the transfer of electrons is mediated by the electrically conductive polymers 13) without the involvement of an oxidation-reduction substance, such as an electron transfer mediator. In cases where an electron transfer mediator is used, if the molecules of the electron transfer mediator are immobilized so as not to be diffused, it is possible to measure the charge transfer limiting current.

As illustrated in FIG. 2, a structure in the detection layer 6 is that molecules of the enzymes 12 are crosslinked by the crosslinking agent 15, and the crosslinked molecules are further entangled intricately by the electrically conductive polymers 13. Electrons generated by enzymatic reaction are able to transfer directly or via the electrically conductive polymers 13 to the working electrode 3. In other words, the enzyme electrode 11 according to the embodiment is configured such that the enzymes 12 transfer and receive the electrons to and from the working electrode 3 by way of the direct electron transfer in the detection layer 6.

The limit distance within which the direct electron transfer could occur in a physiological reaction system is reported to be from 10 to 20 Å. In the electron transfer in an electrochemical reaction system comprising an electrode and an enzyme, the detection of the electron transfer on the electrode will be difficult if the distance between the electrode and the enzyme is longer than the above mentioned limit distance, unless it involves the transfer (for example, transfer by diffusion) of a mediator. Therefore, in the detection layer 6, the active sites of the enzymes 12 (the sites at which electrons are generated due to the enzyme reaction) and the electrically conductive sites of the electrically conductive polymers 13 are located within a distance suitable for electron transfer, in other words, the electrically conductive sites and the active sites are located close enough so that electrons are transferred therebetween in a suitable manner.

### (Enzyme)

Examples of the enzyme 12 include oxidoreductases. Examples of the oxidoreductase include: glucose oxidase (GOD), galactose oxidase, bilirubin oxidase, pyruvic acid oxidase, D- or L-amino acid oxidase, amine oxidase, cholesterol oxidase, choline oxidase, xanthine oxidase, sarcosine oxidase, L-lactic acid oxidase, ascorbic acid oxidase, cytochrome oxidase, alcohol dehydrogenase, glutamate dehydrogenase, cholesterol dehydrogenase, aldehyde dehydrogenase, glucose dehydrogenase (GDH), fructose dehydrogenase, sorbitol dehydrogenase, lactate dehydrogenase, malate dehydrogenase, glycerol dehydrogenase, 17β hydroxysteroid dehydrogenase, estradiol 17β dehydrogenase, amino acid dehydrogenase, glyceraldehyde 3-phosphoric acid dehydrogenase, 3-hydroxysteroid dehydrogenase, diaphorase, cytochrome oxidoreductase, catalase, peroxidase, glutathione reductase, and the like. Among others, the enzyme 12 is preferably a saccharide oxidoreductase. Examples of the saccharide oxidoreductase include: glucose oxidase (GOD), galactose oxidase, glucose dehydrogenase (GDH), fructose dehydrogenase, and sorbitol dehydrogenase.

Further, the oxidoreductase can contain at least one of pyrroloquinoline quinone (PQQ) and flavin adenine dinucleotide (FAD), as a catalytic subunit and a catalytic domain. Examples of the oxidoreductase containing PQQ include PQQ glucose dehydrogenase (PQQGDH). Examples of the oxidoreductase containing FAD include cytochrome glucose dehydrogenase (Cy-GDH) and glucose oxidase (GOD), having an α-subunit containing FAD. In addition, the oxidoreductase can contain an electron transfer subunit or an electron transfer domain. Examples of the electron transfer subunit include a subunit containing heme which has a function of giving and receiving electrons. Examples of the oxidoreductase having the subunit containing heme include those containing cytochrome. For example, a fusion protein of glucose dehydrogenase or PQQGDH with cytochrome can be used.

Further, examples of the enzyme containing the electron transfer domain include cholesterol oxidase and quinoheme ethanol dehydrogenase (QHEDH (PQQ Ethanol dh)). As the electron transfer domain, it is preferred to use a domain containing cytochrome containing heme which has a function of giving and receiving electrons. Examples thereof include "QHGDH" (fusion enzyme; GDH with heme domain of QHGDH)), sorbitol dehydrogenase (Sorbitol DH), D-fructose dehydrogenase (Fructose DH), Glucose-3-Dehydrogenase derived from *Agrobacterium tumefasience* (G3DH from *Agrobacterium tumefasience*)*,* and cellobiose dehydrogenase. A fusion protein of PQQGDH with cytochrome, which is an example of the above mentioned subunit containing cytochrome, and a cytochrome domain of PQQGDH, which is an example of the domain containing cytochrome, are disclosed, for example, in WO 2005/030807. Further, as the oxidoreductase, it is preferred to use an oligomeric enzyme comprising at least a catalytic subunit and a subunit containing cytochrome containing heme which has a function as an electron acceptor.

### (Electrically conductive polymer)

Examples of the electrically conductive polymer 13 include: polypyrrole, polyaniline, polystyrene sulfonate, polythiophene, polyisothianaphthene, polyethylenedioxythiophene(poly(3,4-ethylenedioxythiophene)pol y(styrene sulfonate)), and combinations thereof. Examples of the commercially available product thereof, specifically, examples of the commercially available product of polypyrrole include: "SSPY" (ethyl 3-methyl-4-pyrrolecarboxylate) (manufactured by KAKEN INDUSTRY Co., Ltd.) . Examples of the commercially available product of polyaniline include "AquaPASS 01-x" (manufactured by TA Chemical Co., Ltd.), and the like. Examples of the commercially available product of polystyrene sulfonate include "Poly-NaSS" (manufactured by TOSOH ORGANIC CHEMICAL CO., LTD.). Examples of the commercially available product of polythiophene include "Espacer 100" (manufactured by TA Chemical Co., Ltd.). Examples of the commercially available product of polyisothianaphthene include "Espacer 300" (manufactured by TA Chemical Co., Ltd.)). Examples of the commercially available product of polyethylenedioxythiophene(poly(3,4-ethylenedioxythiophene)pol y(styrene sulfonate)) include "PEDOT-PSS" (manufactured by Polysciences Inc.). Further, as the electrically conductive polymer 13, an electrically conductive polymer having various attributes (for example, water solubility) can be used. It is preferred that the electrically conductive polymer 13 contain a hydroxyl group or a sulfo group as a functional group.

### (Sugar)

The sugar 14 is a sugar which does not serve as a substrate for the enzyme 12. The sugar 14 contains, for example, 1 to 6 constituent sugars, preferably, 2 to 6 constituent sugars. The sugar 14 may be a D-sugar or L-sugar, or a mixture thereof, and these can be used alone or in a combination of two or more. However, in cases where a sugar such as glucose is the target to be measured, a sugar which is different from the sugar to be measured, and which does not serve as a substrate for the enzyme 12 is used as the sugar 14. Examples of disaccharides include: xylobiose, agarobiose, carabiose, maltose, isomaltose, sophorose, cellobiose, trehalose, neotrehalose, isotrehalose, inulobiose, vicianose, isoprimeverose, sambubiose, primeverose, solabiose, melibiose, lactose, lycobiose, epicellobiose, sucrose, turanose, maltulose, lactulose, epigentibiose, robinobiose, silanobiose, rutinose, and the like. Examples of trisaccharides include: glucosyl trehalose, cellotriose, chacotriose, gentianose, isomaltotriose, isopanose, maltotriose, manninotriose, melezitose, panose, planteose, raffinose, solatriose, umbelliferose, and the like. Examples of tetrasaccharides include maltosyl trehalose, maltotetraose, stachyose, and the like. Examples of pentasaccharides include maltotriosyl trehalose, maltopentaose, verbascose, and the like. Examples of hexasaccharides include maltohexaose and the like.

### (Crosslinking agent)

Examples of the crosslinking agent 15, specifically, examples of aldehyde group-containing compounds to be used as the crosslinking agent 15 include: glutaraldehyde, formaldehyde, malonaldehyde, terephthalaldehyde, isobutyraldehyde, valeraldehyde, isovaleraldehyde, cinnamaldehyde, nicotinaldehyde, glyceraldehyde, glycoaldehyde, succinaldehyde, adipaldehyde, isophthalaldehyde, terephthalaldehyde, and the like. Examples of carbodiimide group-containing compounds include hexamethylene diisocyanate, hydrogenated xylylene diisocyanate, xylylene diisocyanate, 2,2,4-trimethyl hexamethylene diisocyanate, 1,12-diisocyanate dodecane, norbornane diisocyanate, 2,4-bis-(8-isocyanate octyl)-1,3-dioctyl cyclobutane, 4,4'-dicyclohexylmethane diisocyanate, tetramethyl xylylene diisocyanate, isophorone diisocyanate, and the like. The carbodiimide group-containing compounds are commercially available under the names of: CARBODILITE V-02, CARBODILITE V-02-L2, CARBODILITE V-04, CARBODILITE V-06, CARBODILITE E-02, CARBODILITE V-01, CARBODILITE V-03, CARBODILITE V-05, CARBODILITE V-07 and CARBODILITE V-09 (manufactured by Nisshinbo Chemical, Inc.). Examples of maleimide group-containing compounds include : m-maleimidobenzoyl-N-hydroxysuccinimide ester, sulfosuccinimidyl 4-(p-maleimidophenyl)butyrate, m-maleimidobenzoyl sulfosuccinimide ester, N-γ-maleimidobutyryloxy succinimide ester, succinimidyl 4-(N-maleimidomethyl)cyclohexane)1-carboxylate, N-succinimidyl-2-maleimidoacetic acid, N-succinimidyl-4-maleimidobutyric acid, N-succinimidyl-6-maleimidohexanoic acid, N-succinimidyl-4-maleimidomethyl cyclohexane-1-carboxylic acid, N-succinimidyl-4-maleimidomethyl cyclohexane-1-carboxylic acid, N-succinimidyl-4-maleimidomethyl benzoic acid, N-succinimidyl-3-maleimidobenzoic acid, N-succinimidyl-4-maleimidophenyl-4-butyric acid, N-succinimidyl-4-maleimidophenyl-4-butyric acid, N,N'-oxydimethylene-dimaleimide, N,N'-o-phenylene-dimaleimide, N,N'-m-phenylene-dimaleimide, N,N'-p-phenylene-dimaleimide, and N,N'-hexamethylene-dimaleimide, N-succinimidyl maleimide carboxylic acid, and the like. As the commercially available product of the maleimide group-containing compound, SANFEL BM-G (manufactured by SANSHIN CHEMICAL INDUSTRY Co., Ltd) can be mentioned. Examples of oxazoline group-containing compounds include oxazoline compounds such as : 2,2'-bis-(2-oxazoline), 2,2'-methylene-bis-(2-oxazoline), 2,2'-ethylene-bis-(2-oxazoline), 2,2'-trimethylene-bis-(2-oxazoline), 2,2'-tetramethylene-bis-(2-oxazoline), 2,2'-hexamethylene-bis-(2-oxazoline), 2,2'-octamethylene-bis-(2-oxazoline), 2,2'-ethylene-bis-(4,4'-dimethyl-2-oxazoline), 2,2'-p-phenylene-bis-(2-oxazoline), 2,2'-m-phenylene-bis-(2-oxazoline), 2,2'-m-phenylene-bis-(4,4'-dimethyl-2-oxazoline), bis-(2-oxazolinylcyclohexane)sulfide, and bis-(2-oxazolinylnorbornane)sulfide. Further, examples of addition polymerizable oxazoline compounds include: 2-vinyl-2-oxazoline, 2-vinyl-4-methyl-2-oxazoline, 2-vinyl-5-methyl-2-oxazoline, 2-isopropenyl-2-oxazoline, 2-isopropenyl-4-methyl-2-oxazoline, 2-isopropenyl-5-ethyl-2-oxazoline, and the like. The compounds obtained by polymerization or copolymerization of one or more of these compounds can also be used. The oxazoline group-containing compounds are commercially available under the names of: Epocros WS-500, Epocros WS-700, Epocros K-1010E, Epocros K-1020E, Epocros K-1030E, Epocros K-2010E, Epocros K-2020E, Epocros K-2030E, Epocros RPS-1005 and Epocros RAS-1005 (all of the above are manufactured by NIPPON SHOKUBAI Co., Ltd.); and NK linker FX (manufactured by SHIN-NAKAMURA CHEMICAL Co., Ltd.). Specific examples of epoxy group-containing compounds include: sorbitol polyglycidyl ether, polyglycerol polyglycidyl ether, diglycerol polyglycidyl ether, glycerol polyglycidyl ether, trimethylolpropane polyglycidyl ether, ethylene glycol diglycidyl ether, polyethylene glycol diglycidyl ether, propylene glycol diglycidyl ether, polypropylene glycol diglycidyl ether, and the like. Two or more kinds of these compounds can be used in combination. Further, the epoxy group-containing compounds are commercially available under the names of: Denacol EX-611, Denacol EX-612, Denacol EX-614, Denacol EX-614B, Denacol EX-512, Denacol EX-521, Denacol EX-421, Denacol EX-313, Denacol EX-314, Denacol EX-321, Denacol EX-810, Denacol EX-811, Denacol EX-850, Denacol EX-851, Denacol EX-821, Denacol EX-830, Denacol EX-832, Denacol EX-841, Denacol EX-861, Denacol EX-911, Denacol EX-941, Denacol EX-920, Denacol EX-145 and Denacol EX-171 (manufactured by Nagase ChemteX Corporation) ; SR-PG, SR-2EG, SR-8EG, SR-8EGS, SR-GLG, SR-DGE, SR-4GL, SR-4GLS and SR-SEP (all of the above are trade names, manufactured by Sakamoto Yakuhin Kogyo Co., Ltd.); and Epolite 200E, Epolite 400E and Epolite 400P (all of the above are manufactured by KYOEISHA CHEMICAL Co., LTD). The type of the crosslinking agent 15 is not limited to the above mentioned compounds and commercially available products. The crosslinking agent 15 may also be a compound containing at least one functional group selected from aldehyde group, maleimide group, carbodiimide group, oxazoline group and epoxy group. The form of the crosslinking agent 15 is not limited, either. The crosslinking agent 15 may be in the form of monomer, polymer or the like.

### (Electrically conductive particles)

It is preferred that the detection layer 6 further include electrically conductive particles. As the electrically conductive particles, particles of a metal such as gold, platinum, silver or palladium; or a higher-order structure made of a carbon material can be used. The higher-order structure can contain, for example, one or more types of fine particles (carbon fine particles) selected from particles of electrically conductive carbon black, Ketjenblack (registered trademark), carbon nanotube (CNT) and fullerene.

Further, the surface of the detection layer 6 maybe covered with an outer-layer film made of cellulose acetate and the like. Examples of raw materials for the outer-layer film, in addition to cellulose acetate, include: polyurethane, polycarbonate, polymethyl methacrylate, butyl methacrylate, polypropylene, polyether ether ketone, and the like.

### (Method of Producing Enzyme Electrode)

The enzyme electrode 11 is, e.g., produced as follows. To be specific, metal layers functioning as the working electrode 3, the counter electrode 4 and the reference electrode 5 are formed on a single surface of the substrate 2. For example, the metallic materials are deposited by Physical Vapor Deposition (PVD, e.g., sputtering) or by Chemical Vapor Deposition (CVD) on the single surface of the film-like substrate 2 having a predetermined thickness (e.g., about 100 µm), thereby forming the metal layers each having a desired thickness (e.g., about 30 nm). In place of the metal layers, it is also feasible to form electrode layers composed of the carbonic materials instanced by carbon, which are deposited by screen deposition.

The working electrode 3, the counter electrode 4 and the reference electrode 5 may be made from the same material, in which case it is feasible to configure the working electrode 3, the counter electrode 4 and the reference electrode 5 in the same steps. For example, when configuring the working electrode 3, the counter electrode 4 and the reference electrode 5 by using carbon, the working electrode 3, the counter electrode 4 and the reference electrode 5 are configured through the same deposition steps, while enabling an omission of a deposition step for configuring a silver-silver chloride electrode. It is therefore feasible to decrease a manufacturing cost of the biosensor 1 by configuring the working electrode 3, the counter electrode 4 and the reference electrode 5 through the same steps.

Next, the detection layer 6 is formed on the working electrode 3. Namely, a solution (reagent) containing the enzymes 12, the electrically conductive polymers 13, the sugars 14 and the crosslinking agent 15 is prepared. Herein, a concentration of the sugars 14 is preferably 0.1 - 2% by weight and more preferably 0.2 - 2% by weight. The solution (reagent) is dropped down to the surface of the working electrode 3. The solution (reagent) is solidified by drying on the working electrode 3, thereby enabling acquisition of the enzyme electrode 11 with the detection layer 6 being configured on the working electrode 3.

By using the enzyme electrode 11 according to the embodiment, the concentration of the substance to be tested contained in a sample can be measured based on the charge transfer limiting current. The measurement target substance is not particularly limited as long as it can be measured by the measuring method using the enzyme electrode 11 of the embodiment. However, the measurement target substance is preferably a substance derived from a living body, which can serve as an index of a disease and/or health status, and examples thereof include glucose, cholesterol, and the like. The sample is not particularly limited as long as it contains the measurement target substance. However, a biological sample, such as blood or urine is preferred.

### (Apparatus)

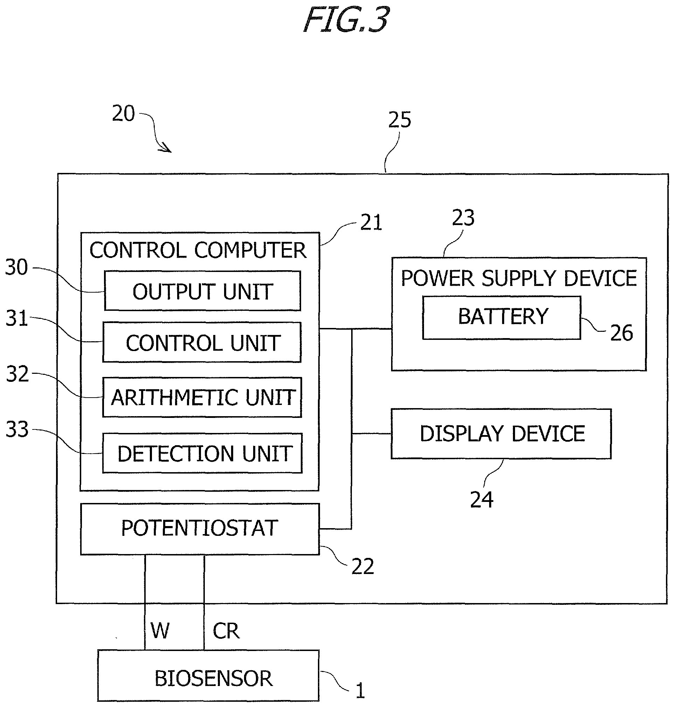

Subsequently, a measurement apparatus according to the embodiment will be described. FIG. 3 is a diagram illustrating one example of a configuration of a measurement apparatus 20 according to the embodiment. FIG. 3 depicts architecture of main electronic components accommodated within the measurement apparatus 20. The measurement apparatus 20 includes a control computer 21, a potentiostat 22, a power supply device 23 and a display device 24. The control computer 21, the potentiostat 22 and the power supply device 23 are provided on a board housed in a housing 25.

The control computer 21 includes hardwarewise a processor instanced by a Central Processing Unit (CPU), a storage device instanced by a memory (Random Access Memory (RAM), Read Only Memory (ROM)), and a communication unit. The processor loads a program stored on the ROM onto the RAM and runs the loaded program, thereby functioning as an apparatus including an output unit 30, a control unit 31, an arithmetic unit 32 and a detection unit 33. Note that the control computer 21 may include an auxiliary storage device instanced by a semiconductor memory (Electrically Erasable Programmable ROM (EEPROM), flash memory) and a hard disk.

The control unit 31 controls the timing for applying the voltage and the value of the voltage to be applied. The power supply device 23 includes a battery 26, and supplies electricity to the control computer 21 and the potentiostat 22 for operation. It is also possible to dispose the power supply device 23 outside the housing 25. The potentiostat 22 is a device which maintains the potential of the working electrode 3 constant with respect to the potential of the reference electrode 5. The potentiostat 22, which is controlled by the control unit 31, applies a predetermined amount of voltage between the working electrode 3 and the counter electrode 4 of the biosensor 1 using terminals CR and W; measures the response current of the working electrode 3 which can be obtained at the terminal W; and send the result of the measurement to the detection unit 33.

The arithmetic unit 32 calculates a concentration of a measurement target substance from a detected electric current value, and stores a calculation result of the concentration of the measurement target substance in the storage device. The output unit 30 performs data communications with the display device 24, and transmits the calculation result of the concentration of the measurement target substance to the display device 24. The display device 24 is capable of displaying the calculation result of the concentration of the measurement target substance on a display screen in a predetermined format.

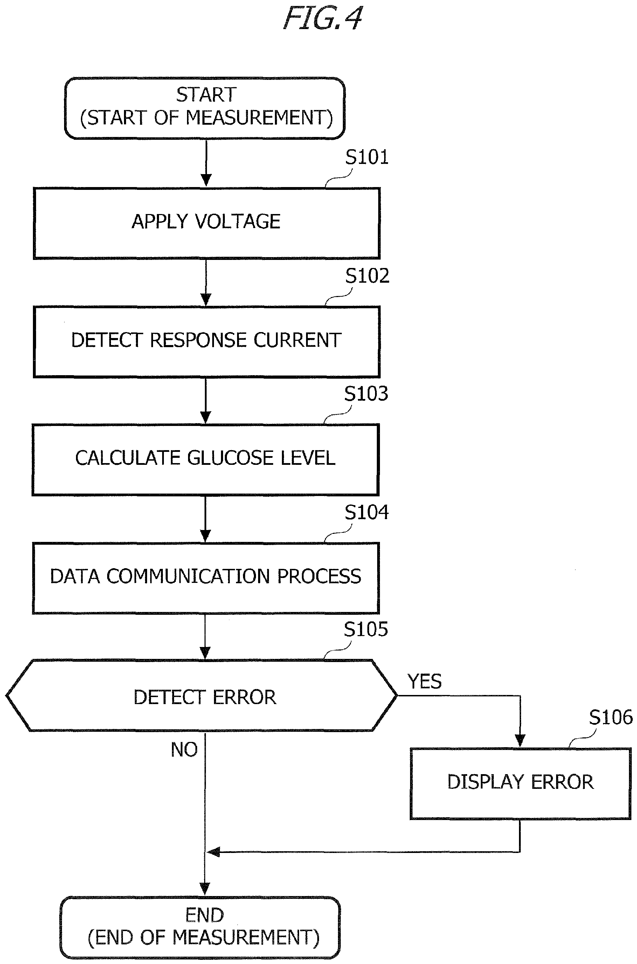

FIG. 4 is a flowchart illustrating one example of a concentration measuring process of the measurement target substance by the control computer 21. The control unit 31, upon accepting an instruction to start measuring the concentration of the measurement target substance, controls the potentiostat 22 to start measuring a response current from the working electrode 3 by applying a predetermined voltage to the working electrode 3 (step S101) . Note that the instruction to start measuring the concentration may also be made as triggered by detecting that the biosensor 1 is attached to the measurement apparatus 20.

Next, the potentiostat 22 measures the response current acquired by applying the voltage, and sends the measured response current value to the detection unit 33 (step S102) . The detection unit 33 thereby detects the response current. The response current is a charge transfer limiting current based on the electron transfer to the electrode, which is derived from the measurement target substance (which is herein glucose) within a sample. The response current is preferably a steady-state current after an elapse of, e.g., 1 - 20 sec since applying the voltage after a transient current has been generated by charging an electric double layer.

The arithmetic unit 32 calculates a glucose level by executing an arithmetic process based on the response current value (step S103). Forexample, the arithmetic unit 32 previously retains a glucose level calculation formula or glucose level calibration curve data, corresponding to glucose dehydrogenase disposed on the working electrode 3. The arithmetic unit 32 calculates the glucose level by using the glucose level calculation formula or the glucose level calibration curve data.

The output unit 30 transmits a calculation result of the glucose level to the display device 24 via a communication link established between the control computer 21 and the display device 24 (step S104) . Thereafter, the control unit 31 detects whether a measurement error occurs or not (step S105) . The control unit 31, when not detecting the error (step S105: NO), finishes the measurement, and displays the glucose level on the display device 24. The processingbased on the processing flow in FIG. 4 is finished. Whereas when detecting the error (step S105: YES), the control unit 31 displays the error on the display device 24 (step S106) . Thereafter, the processing based on the processing flow in FIG. 4 is finished.

The control unit 31 saves the calculation result on the storage device, and may also display the calculation result on the display device 24 by reading the calculation result from the storage device at an arbitrary timing. A user is able to check the calculation result at the arbitrary timing. Note that the control unit 31 detects the measurement error (step S105) after transmitting the calculation result to the display device 24 (step S104), and sequential orders of these steps may also be, however, replaced with each other. The discussion made above has demonstrated the concentration measurement process when the measurement target substance is the glucose, and the embodiment is not, however, limited to this concentration measurement process. The measurement target substance may encompass other substances without being limited to the glucose.

### [Working Example]

A test for measuring the glucose level will hereinafter be described. In a Test 1 and a Comparative Example 1 that follow, the response current values with respect to the samples with the glucose levels being adjusted to 0 mg/dL, 100 mg/dL, 300 mg/dL, 600 mg/dL and 800 mg/dL were measured by using a 3-electrode system (the working electrode, the counter electrode and the reference electrode) . In a Test 2 and a Comparative Example 2 that follow, the response current values with respect to the samples with the glucose levels being adjusted to 0 mg/dL, 100 mg/dL and 600 mg/dL were measured by using the 3-electrode system. Further in the Tests 1, 2 and the comparative Examples 1, 2, the response current values were measured based on a chronoamperometry method of applying a potential difference of 200 mV to the electrode system at a temperature of 26 °C (±1°C) by using the 3-electrode system.

### <Test 1>

The following are the materials of the respective electrodes in Test 1.
- Working electrode (WE) : carbon (the planar dimension was 0.5 mm²)
- Counter electrode (CE) : carbon (the planar dimension was 4.8 mm²)
- Reference electrode (RE): carbon (the planar dimension was 0.5 mm²)

### <Comparative Example 1>

The following are the materials of the respective electrodes in Comparative Example 1.
- Working electrode (WE) : carbon (the planar dimension was 0.5 mm²)
- Counter electrode (CE) : carbon (the planar dimension was 4.8 mm²)
- Reference electrode (RE): silver-silver chloride (the planar dimension was 0.5 mm²).

### <Measurement Result>

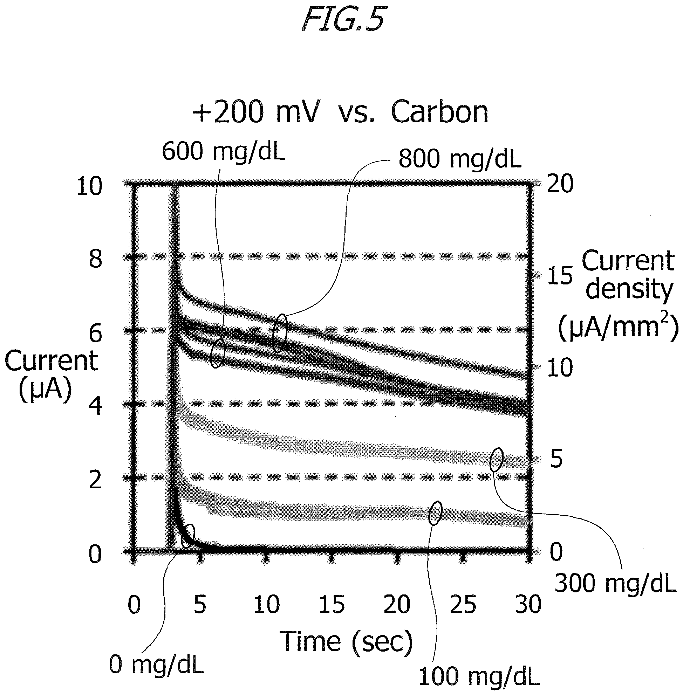

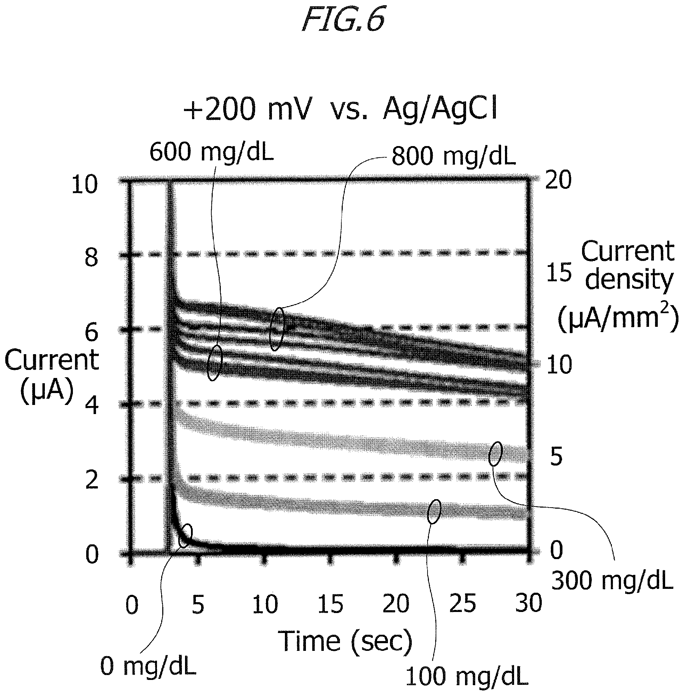

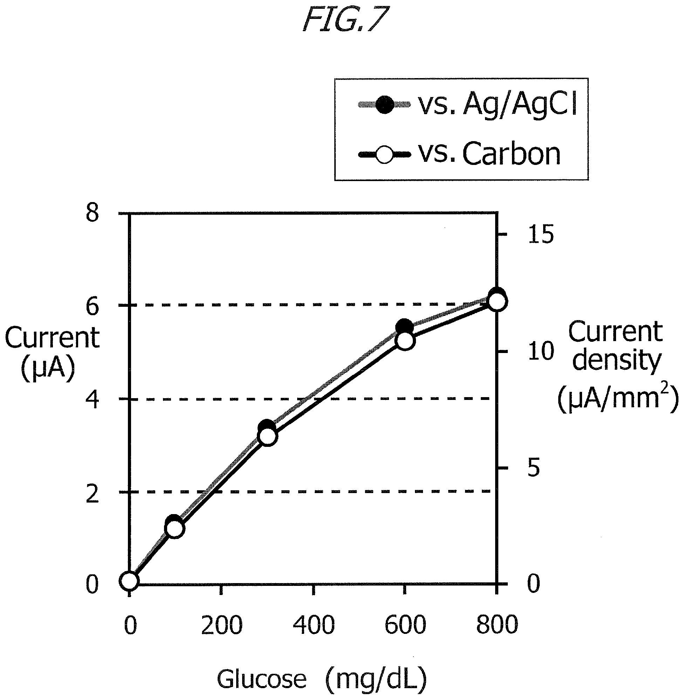

FIG. 5 is a graph indicating a measurement result of Test 1. FIG. 6 is a graph indicating a measurement result of Comparative Example 1. In FIGS. 5 and 6, variations with a passage of time (time course) of the response current values are graphed. FIG. 7 is a graph indicating the measurement results of Test 1 and Comparative Example 1. In FIG. 7, with respect to the measurement results of Test 1 and Comparative Example 1, the response current values were plotted per glucose level after an elapse of 8 sec (after an elapse of 5 sec since reaching a current peak) since starting applying the voltage. As depicted in FIGS. 5 through 7, when the material of the reference electrode was carbon, there were measured the same response current values as when the material of the reference electrode was silver-silver chloride.

### <Test 2>

The following are the materials of the respective electrodes in Test 2.
- Working electrode (WE) : carbon (the planar dimension was 0.5 mm²)
- Counter electrode (CE) : carbon (the planar dimension was 4.8 mm²) Reference electrode (RE) : gold (the planar dimension was 0.5 mm²)

### <Comparative Example 2>

The following are the materials of the respective electrodes in Comparative Example 2.
- Working electrode (WE) : carbon (the planar dimension was 0.5 mm²)
- Counter electrode (CE) : carbon (the planar dimension was 4.8 mm²)
- Reference electrode (RE): silver-silver chloride (the planar dimension was 0.5 mm²).

### <Measurement Result>

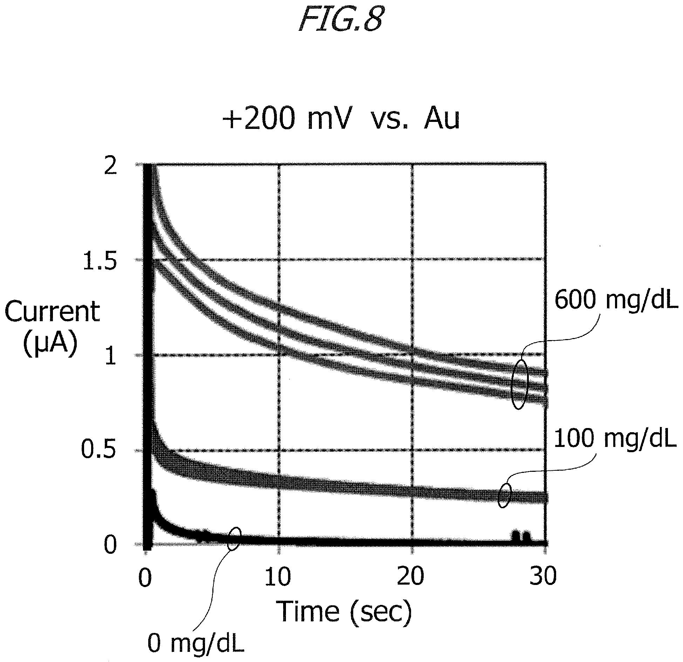

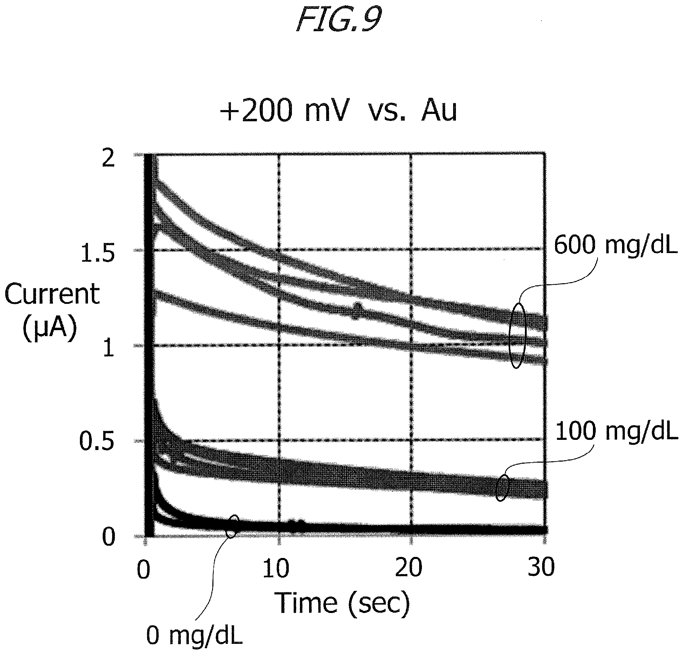

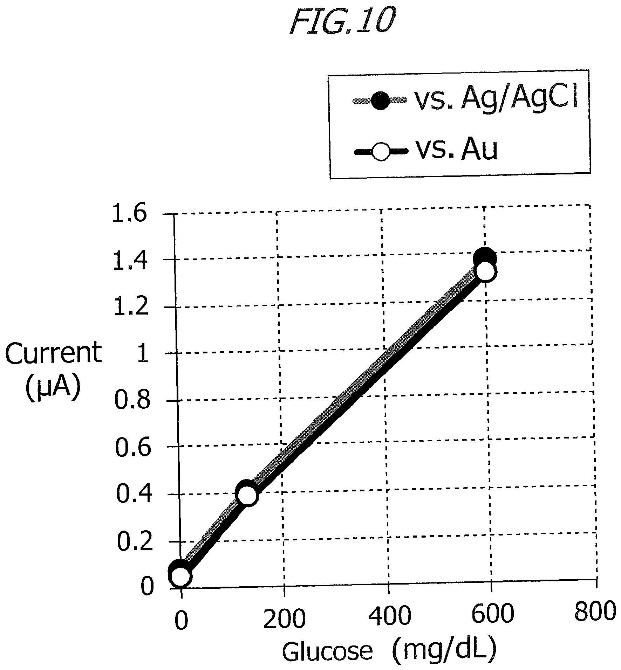

FIG. 8 is a graph indicating a measurement result of Test 2. FIG. 9 is a graph indicating a measurement result of Comparative Example 2. In FIGS. 8 and 9, the variations with the passage of time (time course) of the response current values are graphed. FIG. 10 is a graph indicating the measurement results of Test 2 and Comparative Example 2. In FIG. 10, with respect to the measurement results of Test 2 and Comparative Example 2, the response current values were plotted per glucose level after an elapse of 5 sec since starting applying the voltage. As depicted in FIGS. 8 through 10, when the material of the reference electrode was gold, there were measured the same response current values as when the material of the reference electrode was silver-silver chloride.

Test 1 demonstrated the instance in which the material of the reference electrode was carbon, and Test 2 demonstrated the instance in which the material of the reference electrode was gold.

## Claims

1. A biosensor (1) comprising:
a working electrode (3);
a counter electrode (4);
a reference electrode (5); and
a detection layer (6) contacting the working electrode and containing a crosslinking agent, an electrically conductive polymer, and an enzyme transferring and receiving electrons to and from the working electrode,
the reference electrode being a polarizable electrode which is carbon or gold.

2. The biosensor according to claim 1, wherein the working electrode and the counter electrode are polarizable electrodes, and
the working electrode, the counter electrode and the reference electrode are made from the same material.

## Patentansprüche

1. Biosensor (1), umfassend:
eine Arbeitselektrode (3);
eine Gegenelektrode (4);
eine Referenzelektrode (5); und
eine Detektionsschicht (6), die mit der Arbeitselektrode in Kontakt steht und ein Vernetzungsmittel, ein elektrisch leitfähiges Polymer und ein Enzym enthält, das Elektronen an die Arbeitselektrode überträgt und von der Arbeitselektrode aufnimmt,
wobei die Referenzelektrode eine polarisierbare Elektrode ist, die aus Kohlenstoff oder Gold besteht.

2. Biosensor nach Anspruch 1, wobei die Arbeitselektrode und die Gegenelektrode polarisierbare Elektroden sind, und
die Arbeitselektrode, die Gegenelektrode und die Referenzelektrode aus dem gleichen Material hergestellt sind.

## Revendications

1. Biocapteur (1) comprenant :
une électrode de travail (3) ;
une contre-électrode (4) ;
une électrode de référence (5) ; et
une couche de détection (6) venant en contact avec l'électrode de travail et contenant un agent de réticulation, un polymère électroconducteur et une enzyme transférant des électrons à l'électrode de travail et recevant des électrons de celle-ci,
l'électrode de référence étant une électrode polarisable qui est en carbone ou en or.

2. Biocapteur selon la revendication 1, dans lequel l'électrode de travail et la contre-électrode sont des électrodes polarisables, et
l'électrode de travail, la contre-électrode et l'électrode de référence sont faites du même matériau.
